# EUROPÄISCHE PATENTSCHRIFT

(11) **EP 1 793 739 B1**
(45) Veröffentlichungstag und Bekanntmachung des Hinweises auf die Patenterteilung: **10.03.2010**
(21) Anmeldenummer: 05759695.9
(22) Anmeldetag: 12.07.2005
(51) Int. Cl.: A61B 6/14

(54) **VERFAHREN ZUR BESTIMMUNG DER SOLL-RELATIVLAGE EINES PATIENTEN IN EINEM DENTALEN PANORAMA-RÖNTGENGERÄT BZW. DER SOLL-BAHN, AUF WELCHER DIESES BEZÜGLICH EINES PATIENTEN BEWEGT WIRD SOWIE EINE DAFÜR GEEIGNETE VORRICHTUNG**
METHOD FOR DETERMINING THE SET RELATIVE POSITION OF A PATIENT IN A DENTAL PANORAMA X-RAY APPARATUS OR THE SET PATH ON WHICH THIS APPARATUS IS MOVED WITH REGARD TO A PATIENT, AND A DEVICE SUITED THEREFOR
PROCEDE POUR DETERMINER LA POSITION RELATIVE THEORIQUE D'UN PATIENT DANS UN APPAREIL RADIOGRAPHIQUE PANORAMIQUE DENTAIRE ET LA TRAJECTOIRE THEORIQUE SELON LAQUELLE LEDIT APPAREIL DOIT ETRE DEPLACE PAR RAPPORT A UN PATIENT ET DISPOSITIF APPROPRIE A CET EFFET

(30) Priorität: 27.08.2004 DE 102004041440
(43) Veröffentlichungstag der Anmeldung: 13.06.2007
(73) Patentinhaber: Dürr Dental AG, 74321 Bietigheim-Bissingen (DE)
(72) Erfinder: THOMS, Michael, 74321 Bietigheim-Bissingen (DE)
(74) Vertreter: Ostertag, Reinhard
(86) Internationale Anmeldenummer: PCT/EP2005/007510
(87) Internationale Veröffentlichungsnummer: WO 2006/024342

(56) Entgegenhaltungen:
- WO-A-02/065918
- DE-A1- 3 808 009
- US-A- 4 229 656
- US-A- 4 782 503
- US-A- 5 195 114
- US-A- 5 692 027

## Beschreibung

Die Erfindung betrifft ein Verfahren zur Bestimmung der Soll-Relativlage eines Patienten in einem dentalen Panorama-Röntgengerät, bei welchem
a) die Krümmung eines vorderen Bereichs eines Zahnbogens eines Patienten ermittelt wird; und
b) daraus und aus einer Projektionsbogenfläche des Panorama-Röntgengeräts Soll-Lagekoordinaten für den Kiefer des Patienten berechnet werden.

Ferner betrifft die Erfindung ein Verfahren zur Bestimmung der Soll-Bahn, auf welcher ein dentales Panorama-Röntgengerät bezüglich eines Patienten bewegt wird, bei welchem
a) die Krümmung eines vorderen Bereichs eines Zahnbogens eines Patienten ermittelt wird; und
b) der Zahnbogen des Patienten an einer Stelle im Panorama-Röntgengerät fixiert wird, die innerhalb des von einer Röntgenstrahlungsquelle und einer starren mit dieser bewegten Detektoreinheit überstrichenen Raumes liegt.

Außerdem betrifft die Erfindung eine Vorrichtung zur Bestimmung der Soll-Relativlage eines Patienten in einem dentalen Panorama-Röntgengerät bzw. der Soll-Bahn einer Diagnostikeinheit bezüglich des in einem Panorama-Röntgengerät fixierten Kiefers eines Patienten mit
a) einer Sensoreinheit, welche mit Zähnen des Patienten im vorderen Bereich des Kiefers zusammenarbeitet, welche ein für die Lage der Zähne des Patienten charakteristisches Kieferform-Signal bereitstellt;
b) einer Recheneinheit, welche mit dem Kieferform-Signal der Sensoreinheit beaufschlagt ist und aus diesem und der Bahn einer Röntgenstrahlungsquelle und einer Detektoreinheit des Panorama-Röntgengerätes, die die Projektionsbogenfläche des Panorama-Röntgengeräts bestimmt, Soll-Lagekoordinaten für den Kiefer des Patienten berechnet.

Eine Projektionsbogenfläche (kurz: Projektionsbogen) beschreibt diejenige Bogenfläche, welche in dem resultierenden Röntgenbild eines Panorama-Röntgengerätes scharf abgebildet ist. Dies bedeutet, das Röntgenbild zeigt dasjenige Gewebe eines Patienten, welches während der Röntgenaufnahme in der Projektionsbogenfläche liegt.

Bei der Aufnahme eines dentalen Panorama-Röntgenbildes befindet sich der Kopf eines Patienten zwischen einer Röntgenstrahlungsquelle und einem Röntgenstrahlungsdetektor, welche den Kopf des Patienten in einer bogenförmigen Bahn umfahren. Dazu werden die Röntgenstrahlungsquelle und die Detektoreinheit in der Regel in konstantem Abstand zueinander in einer horizontalen Ebene um eine vertikale Drehachse verdreht, welche ihrerseits während des Drehvorganges entlang einer bestimmten Bahn verfahren wird.

Der ideale Verlauf dieser Bahn ergibt sich aus der Zahnbogen-Geometrie eines Patienten. Der Zahnbogen weist im Bereich der vorderen Zähne eine starke Krümmung auf, während die Krümmung im Bereich der mittleren bzw. hinteren Zähne schwächer ist.

Während des Verfahrens der Röntgenstrahlungsquelle und der Detektoreinheit um den Kopf des Patienten herum wird eine Vielzahl von Einzelaufnahmen angefertigt, welche durch eine entsprechende Rechnereinheit zu einem Gesamtbild zusammengefügt werden.

Jede Einzelaufnahme ist ein schmaler, ebener Projektionsbereich zuzuordnen, in dem das von der Röntgenstrahlung durchdrungene Gewebe des Patienten scharf abgebildet ist. Für das Panorama-Röntgenbild werden also, einfach ausgedrückt, jeweils relativ schmale horizontale Bereiche von Einzelbildern zusammengefügt, wobei das Bewegen der Drehachse von Röntgenstrahlungsquelle und Detektoreinheit gemäß einer Standard-Kieferform erfolgt.

Die Projektionsbogenfläche des Panorama-Röntgengerätes ergibt sich aus den entsprechend zusammengefügten horizontalen Bereichen der Projektionsebenen der jeweiligen Einzelbilder bzw. Bildstreifen. Insgesamt resultiert die Projektionsbogenfläche des Panorama-Röntgengerätes aus der Bahn, in der die Röntgenstrahlungsquelle und die Detektoreinheit den Kopf des Patienten umfahren und die in der Regel manuell oder anhand von Daten aus einer Datenbank eingestellt wird.

Zusätzlich sind noch Aufnahme-Einstellungen des dentalen Panorama-Röntgengeräts vorzunehmen, zu denen u.a. die Röhrenspannung, die Belichtungszeit, der Strahlstrom und der Strahlquerschnitt als Parameter für die Röntgenstrahlungsquelle zählen.

Damit das resultierende Röntgenbild auch die gewünschten Bereiche des Patienten zeigt, muß dieser in einer bestimmten Soll-Relativlage in dem Panorama-Röntgengerät positioniert werden, in der der Zahnbogen des Patienten in einem möglichst großen Bereich mit der Projektionsbogenfläche zur Deckung gelangt.

Neben der Einstellung des Panorama-Röntgengerätes erfolgt auch die Positionierung des Patienten üblicherweise manuell. Dazu verfügt das Panorama-Röntgengerät über einen Aufbeißhalter, in welchen der Patient hineinbeißt. Daraufhin wird der Patient, meist mit Hilfe einer optischen Vorrichtung, so eingerichtet, daß die Ebene zwischen seinem Ober- und seinem Unterkiefer horizontal verläuft. Dann wird der Projektionsbogen entsprechend dem von dem Bedienpersonal visuell erfassten Zahnbogen des Patienten manuell eingestellt, und dieser wird zwischen der Röntgenstrahlungsquelle und dem Röntgenstrahlungsdetektor vertikal und horizontal in eine solche Position gebracht, daß die Projektionsbogenfläche des Panorama-Röntgengerätes so gut wie möglich durch die Zahnwurzeln des Patienten verläuft.

Die oben genannten manuellen Einstellungen sind zum einen sehr zeitaufwendig und zum anderen recht fehlerträchtig. Mehr als die Hälfte von erstellten Panorama-Röntgenaufnahmen sind unzureichend, da die Positionsbogenfläche nicht wie gewünscht durch die Zahnwurzeln verläuft und diese daher unscharf abgebildet werden.

Eine Verringerung der Fehlerquellen wurde durch eine Vorrichtung der eingangs genannten Art erzielt, wie sie in der DE 38 08 009 C2 beschrieben ist. Bei dieser wird mittels verschiedener Sensortypen die Position der Schneidezähne und/oder der Eckzähne eines Patienten ermittelt. Die Relativpositionen der erfassten Zähne zueinander dienen als Grundlage der Bestimmung der Soll-Relativlage des Patienten in dem Panorama-Röntgengerät. Um den Zahnbogen des Patienten zu bestimmen, wird auf eine Datenbank zurückgegriffen, in welcher physiologische Patientendaten abgespeichert sind. Aus dieser Datenbank wird dem zu untersuchenden Patienten eine Zahnbogengeometrie zugeordnet, welche eine möglichst große Übereinstimmung mit der aus den Positionen der Schneide- und/oder Eckzähne des zu untersuchenden Patienten ermittelten Zahnbogenform aufweist.

Da zur Bestimmung des Zahnbogens des Patienten lediglich die Schneide- und/oder Eckzähne des Patienten erfaßt werden, kann dem Patienten eine von seiner tatsächlichen Zahnbogenform abweichende Zahnbogen-Geometrie aus der Datenbank zugeordnet werden, welche vor allem im mittleren und hinteren Kieferbereich einen anderen Verlauf nimmt als dies bei dem Patienten tatsächlich der Fall ist.

Demzufolge kann es vorkommen, daß das aufgenommene Röntgenbild den Bereich der Backen- und Mahlzähne des Patienten nicht zufriedenstellend scharf darstellt und für eine anschließende Diagnose durch den behandelnden Zahnarzt ungeeignet ist.

Aufgabe der vorliegenden Erfindung ist es, Verfahren sowie eine Vorrichtung der eingangs genannten Art bereitzustellen, bei welchen die Soll-Relativlage eines Patienten in einem dentalen Panorama-Röntgengerät bzw. die Soll-Bahn, auf welcher ein dentales Panorama-Röntgengerät bezüglich eines Patienten bewegt wird, besser ermittelbar ist.

Dies wird bezogen auf die Soll-Relativlage verfahrensgemäß dadurch gelöst, daß
c) zusätzlich die Krümmung eines mittleren Bereichs und/oder hinteren Bereichs des Zahnbogens des Patienten ermittelt wird und bei der Berechnung der Soll-Lagekoordinaten für den Kiefer berücksichtigt wird.

Bezogen auf die Soll-Bahn, auf welcher ein dentales Panorama-Röntgengerät bezüglich eines Patienten bewegt wird, wird die oben genannte Aufgabe dadurch gelöst, daß
c) zusätzlich die Krümmung eines mittleren Bereichs und/oder hinteren Bereichs des Zahnbogens des Patienten ermittelt wird; und
d) aus den gemessenen Krümmungen des vorderen und mittleren und/oder hinteren Bereichs des Zahnbogens eine Soll-Bahnkurve für die durch Röntgenstrahlungsquelle und Detektoreinheit gebildete Diagnostikeinheit berechnet wird.

Was die Vorrichtung angeht, so wird die genannte Aufgabe dadurch gelöst, daß
c) die Sensoreinheit zusätzlich mit den Zähnen des Patienten im mittleren Bereich und/oder hinteren Bereich des Kiefers zusammenarbeitet.

Durch die zusätzliche Ermittlung der Krümmung des mittleren und/oder hinteren Bereichs des Zahnbogens des Patienten bzw. dadurch, daß die Sensoreinheit mit dem mittleren und/oder dem hinteren Bereich des Kiefers zusammenarbeitet, ist die Bestimmung der Gesamt-Zahnbogen-Geometrie des Patienten mit hoher Genauigkeit möglich. Dadurch kann bei der Berechnung der Soll-Lagekoordinaten des Patienten auf das Zurückgreifen auf in einer Datenbank gespeicherte physiologische Patientendaten verzichtet werden. Auch ist eine fehlerbehaftete visuelle Bestimmung der Zahnbogen-Geometrie des Patienten nicht mehr nötig. Die Berechnung der Soll-Relativlage des Patienten erfolgt auf der Grundlage einer gemessenen Zahnbogen-Geometrie des Patienten, welche mit der tatsächlich vorliegenden Zahnbogen-Geometrie in hohem Maße übereinstimmt. Dadurch fällt die berechnete Soll-Relativlage des Patienten so aus, daß die Projektionsbogenfläche des Panorama-Röntgengerätes bei der Aufnahme des Panorama-Röntgenbildes mit guter Genauigkeit mit dem Zahnbogen des Patienten zur Deckung gelangt.

Durch die Berechnung der Soll-Bahnkurve für die durch Röntgenstrahlungsquelle und Detektoreinheit gebildete Diagnostikeinheit anhand der gemessenen Krümmungen des vorderen und mittleren und/oder hinterem Bereichs gelingt es, die Projektionsbogenfläche an die tatsächliche Zahnbogen-Geometrie des Patienten anzupassen.

Vorteilhafte Ausgestaltungen der erfindungsgemäßen Vorrichtung sind in den abhängigen Ansprüchen beschrieben.

Nachfolgend werden Ausführungsbeispiele der Erfindung anhand der beigefügten Zeichnung erläutert. In dieser zeigen:
- Figur 1: eine Seitenansicht eines dentalen Panorama-Röntgengerätes mit einem ersten Ausführungsbeispiel einer Vorrichtung zur Bestimmung der Soll-Relativlage eines Patienten bzw. der Soll-Bahn einer Diagnostikeinheit;
- Figur 2A und 2B: zwei Beispiele für unterschiedliche Zahnbogen-Geometrien eines Patienten sowie schematisch die Bahn der Drehachse von Röntgenstrahlungsquelle und Detektoreinheit;
- Figur 3A und 3B: schematisch die Veränderung der Relativlage des Patienten bezogen auf die Projektionsbogenfläche des Panorama-Röntgengerätes nach Figur 1;
- Figur 4A und 4B: schematisch die Anpassung des Projektionsbogenfläche des Panorama-Röntgengerätes an die Zahnbogen-Geometrie des Patienten;
- Figur 5: eine Aufsicht auf eine Sensoreinheit der Vorrichtung nach Figur 1;
- Figur 6: eine Seitenansicht eines dentalen Panorama-Röntgengerätes mit einem zweiten Ausführungsbeispiel einer Vorrichtung nach Figur 1 mit einer abgewandelten Sensoreinheit;
- Figur 7: eine Aufsicht auf die Sensoreinheit der Vorrichtung nach Figur 6;
- Figur 8: eine weiter abgewandelte Sensoreinheit eines dritten Ausführungsbeispieles einer Vorrichtung entsprechend Figur 1; und
- Figur 9: eine schematische Darstellung einer Vorrichtung zur Bestimmung der Soll-Bahnkurve einer Diagnostikeinheit eines Panorama-Röntgengeräts.

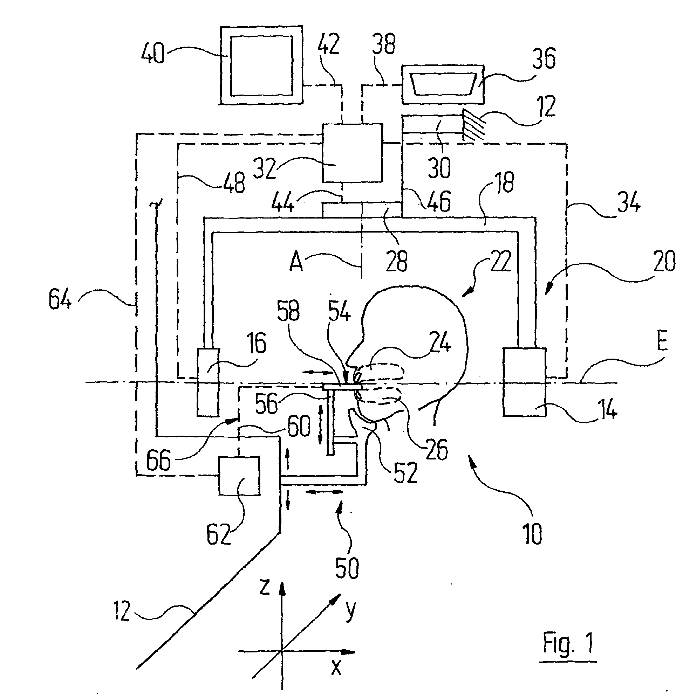

Figur 1 zeigt ein dentales Panorama-Röntgengerät 10 mit einem Rahmen 12, welches eine Röntgenstrahlungsquelle 14 und eine Detektoreinheit in Form eines CCD-Sensors 16 umfaßt. Diese sind um eine vertikale Achse A drehbar und über einen Dreharm 18 starr miteinander verbunden, wobei sie sich in gleicher Höhe gegenüberliegen. Mit E ist eine Aufnahmeebene bezeichnet, welche senkrecht auf der Drehachse A steht und die Mitte des CCD-Sensors 16 sowie die Mitte des auf gleicher Höhe liegenden Fensters der Röntgenstrahlungsquelle 14 schneidet. Ein in Figur 1 angedeutetes x-, y-, z-Koordinatensystem ist rahmenfest. Seine z-Achse verläuft parallel zur Drehachse A, seine x-y-Ebene ist parallel zur Aufnahmeebene E. Die Röntgenstrahlungsquelle 14, der CCD-Sensor 16 und der Dreharm 18 bilden zusammen eine Diagnostikeinheit 20.

Ein Patient, von dessen Kiefer eine Panorama-Röntgenaufnahme erstellt werden soll, wird für die Aufnahme derart in Höhe der Röntgenstrahlungsquelle 14 bzw. des CCD-Sensors 16 positioniert, daß sein Kopf 22, genauer sein Oberkiefer 24 und sein Unterkiefer 26 von der von der Röntgenstrahlungsquelle 14 erzeugten Röntgenstrahlung durchdrungen wird, bevor diese auf den CCD-Sensor 16 trifft.

Der Dreharm 18 ist oberhalb des Patienten um die vertikale Drehachse A drehbar gelagert und mittels eines Antriebsmotors 28 verdrehbar. Dabei ist die Drehachse A zusätzlich horizontal in mindestens einer, vorzugsweise zwei unabhängigen Richtungen, durch einen Koordinaten-Antrieb 30 verfahrbar. Die Röntgenstrahlungsquelle 14 und der CCD-Sensor 16 umfahren somit bei der Erstellung einer Panorama-Röntgenaufnahme den Kopf 22 des Patienten auf einer bestimmten gekrümmten Bahn, welche in der horizontalen Ebene E liegt.

Die Aufnahme-Einstellungen wie die Röhrenspannung, Belichtungszeit, Strahlstrom oder Strahlquerschnitt werden der Röntgenstrahlungsquelle 14 von einer Recheneinheit 32 über eine Datenübertragungsleitung 34 übermittelt.

Die Bahn der Drehachse A und die Aufnahme-Einstellungen der Röntgenstrahlungsquelle 14 können der Recheneinheit 32 über ein Tastenfeld 36 eingegeben werden, welches mittels einer Datenübertragungsleitung 38 mit der Recheneinheit 32 kommuniziert. Die eingegebenen Befehle werden auf einem Anzeigemonitor 40 dargestellt, welcher über eine Datenübertragungsleitung 42 entsprechende Signale von der Recheneinheit 32 erhält.

Die Recheneinheit 32 steuert über eine Leitung 44 den Antriebsmotor 28 und über eine Leitung 46 den Koordinaten-Antrieb 30.

Außerdem empfängt die Recheneinheit 32 über eine Datenübertragungsleitung 48 die Ausgangssignale des CCD-Sensors 16 und übermittelt gegebenenfalls Steuerbefehle an denselben wie Takten des Auslesens, Integrationszeit und Löschen.

Insgesamt dient die Recheneinheit 32 als zentrale Steuereinheit, welche die Aufnahme des Panoramabildes koordiniert, die von dem CCD-Sensor 16 empfangenen Signale aufarbeitet und diese in ein sichtbares Bild umwandelt, welches auf dem Monitor 40 dargestellt wird.

Während der Aufnahme wird der Kopf 22 des Patienten durch eine Positioniervorrichtung 50 fixiert und vor der Röntgenaufnahme in eine entsprechende Soll-Relativlage zu dem Panorama-Röntgengerät 10 gebracht.

Die Positioniervorrichtung 50 umfaßt eine Kinnauflage 52, welche vertikal und in zwei unabhängigen horizontalen Richtungen verfahrbar am Rahmen 12 angebracht ist und nach Erreichen einer gewünschten horizontalen bzw. vertikalen Position fixiert werden kann.

Ferner umfaßt die Positioniervorrichtung 50 einen Aufbeißhalter 54, welcher mit der Kinnauflage 52 über einen vertikal einstellbaren und in einer gewünschten Position fixierbaren Tragstab 56 verbunden ist und welcher selbst wieder in der horizontalen Ebene entlang einer geradlinigen Bahn verfahrbar und dort in einer gewünschten Position fixierbar ist.

Die Verschiebbarkeit des Aufbeißhalters 54 und des Tragstabes 56 dient dabei der Anpassung der Relativ-Positionen der einzelnen Komponenten 52, 54 und 56 an die Physiologie des Patienten.

Die Positionen des Aufbeißhalters 54 und des Tragstabes 56 werden zunächst manuell von Bedienpersonal so eingestellt, daß der Patient auf den Aufbeißhalter 54 beißen kann, während sein Kinn auf der Kinnauflage 52 ruht.

Dann wird die Vertikalposition der Positioniervorrichtung 50 insgesamt so eingestellt, daß der Kieferbereich 24, 26 des Patienten in Höhe der Röntgenstrahlungsquelle 14 und des CCD-Sensors 16 liegt.

Mittels bekannter Positionierhilfen, meist optischer Systeme, wird dafür Sorge getragen, daß die Ebene zwischen Oberkiefer 24 und Unterkiefer 26 des Patienten horizontal verläuft.

Insgesamt ist die Positioniervorrichtung 50 also derart ausgebildet, daß sie in allen notwendigen Richtungen auf die individuelle Kopf- und Kiefer-Physiologie eines Patienten einstellbar ist, wodurch dessen Kieferregion 24, 26 in einer bestimmten Relativ-Position bezüglich des Rahmens 12 fixiert werden kann.

Auf dem Aufbeißhalter 54 ist ein Aufbeißsensor 58 angeordnet. Dieser steht über eine Leitung 60 mit einem Kieferform-Erkennungskreis 62 in Verbindung, der aus dem Ausgangssignal des Aufbeißsensors 58 ein Kieferform-Signal erzeugt und über eine Datenübertragungsleitung 64 an die Recheneinheit 32 übermittelt. Der Aufbeißsensor 58 und der Kieferform-Erkennungskreis 62 bilden zusammen eine Kieferform-Sensoreinheit 66.

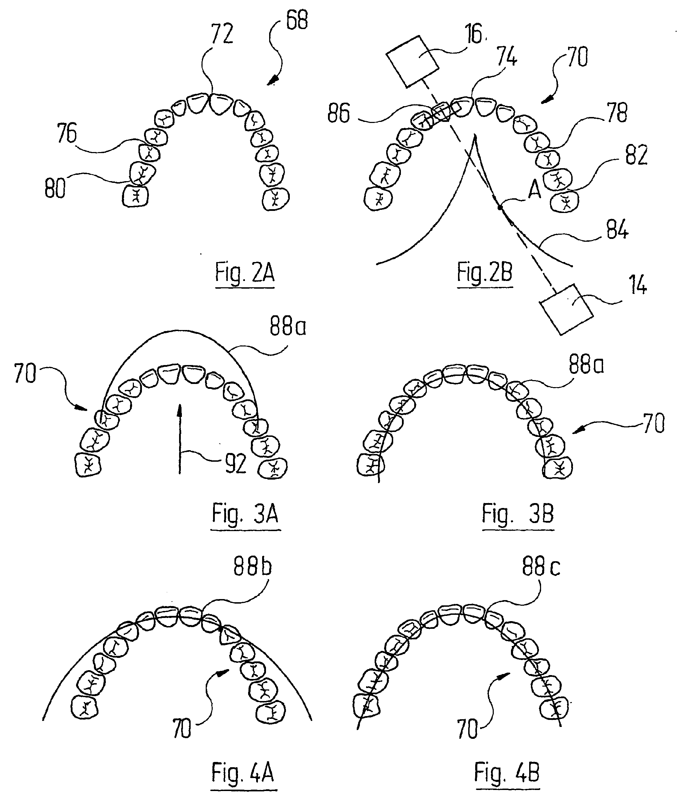

Je nach Physiologie des Kiefers eines Patienten verlaufen die Zahnreihen verschiedener Patienten in unterschiedlichen Zahnbögen. In Figur 2 sind als Beispiel verschiedener Zahnbogen-Geometrien in Figur 2A ein relativ enger Zahnbogen 68 einerseits und in Figur 2B ein relativ weiter Zahnbogen 70 andererseits anhand jeweils einer Oberkiefer-Zahnreihe dargestellt.

Wie dort zu erkennen ist, weisen die Zahnbögen 68, 70 im vorderen Bereich 72 bzw. 74 eine gegenüber einem jeweils mittleren Bereich 76 bzw. 78 und hinterem Bereich 80 bzw. 82 eine stärkere Krümmung auf. Der mittlere Bereich 76, 78 hat demgegenüber eine schwächere Krümmung und der hintere Bereich 80, 82 eine nur geringe Krümmung.

Bei der Drehung des Dreharmes 18 wird dessen vertikale Drehachse A synchron auf einer gekrümmten Bahn verfahren, welche schematisch in Figur 2B am Beispiel des Zahnbogens 70 veranschaulicht und mit den Bezugszeichen 84 versehen ist. Die Bahn 84 der Drehachse A entspricht in etwa einem nach unten offenen V mit konvexen Schenkeln. Durch diesen Verlauf, der je nach Zahnbogenform anders ausfallen sollte, wird gewährleistet, daß die Projektionsebene 86 eines jeden Einzelbildes innerhalb des Zahnbogens 68 bzw. 70 eines Patienten liegt, da der Abstand zwischen Röntgenstrahlungsquelle 14 und CCD-Sensor 16 konstant ist.

Die Figuren 3A und 3B sowie 4A und 4B zeigen Projektionsbogenflächen 88a, 88b und 88c im Vergleich zu dem Zahnbogen 70 von Figur 2B. Damit eine möglichst große Überlappung der Projektionsbogenfläche 88 und dem Zahnbogen 70 des Patienten erreicht wird, ist es zunächst nötig, die tatsächliche Zahnbogengeometrie des zu untersuchenden Patienten zu ermitteln. Dies geschieht mittels des Aufbeißsensors 58.

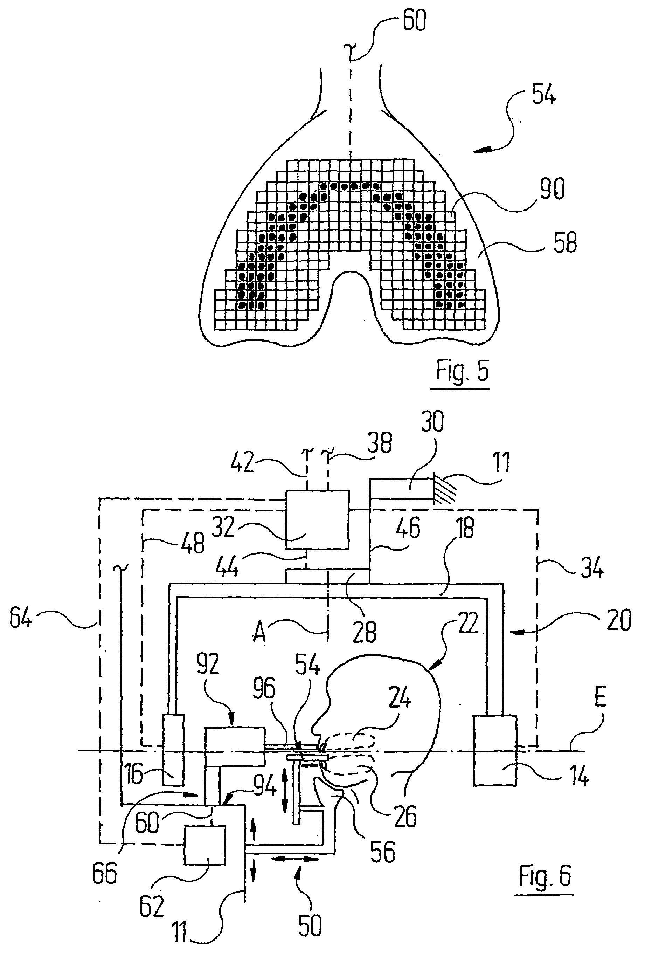

Figur 5 zeigt den Aufbeißsensor 58 bei einer Aufsicht auf die dem Oberkiefer 24 des Patienten zugewandten Seite.

Der Aufbeißsensor 58 umfaßt flächig auf ihm angeordnete Druckfühler, von denen in Figur 5 einer mit der Bezugsziffer 90 gekennzeichnet ist. Beißt ein Patient auf den Aufbeißhalter 54 und somit auf den auf diesem angeordneten Aufbeißsensor 58, wird entsprechend dem Zahnbogen des Patienten eine Vielzahl von Druckfühlern 90 niedergedrückt.

In Figur 5 ist durch die geschwärzten Druckfühler 90 dargestellt, welche Druckfühler 90 näherungsweise niedergedrückt wären, würde ein Patient mit einem Zahnbogen 70, wie in den Figuren 2d, 3 und 4 dargestellt, auf den Aufbeißsensor 58 beissen.

Jeder niedergedrückte Druckfühler 90 erzeugt ein Signal, welches über die Kommunikationsleitung 60 dem Kieferform-Erkennungskreis 62 übermittelt wird. Dieser wiederum erzeugt ein dem Gesamtbild der niedergedrückten Druckfühler 90 entsprechendes Kieferform-Signal.

Das oben zu den Oberkieferzähnen eines Patienten niedergedrückten Druckfühlern 90 des Aufbeißsensors 58 Gesagte gilt sinngemäß entsprechend für die Unterkieferzähne eines Patienten. Dazu umfaßt der Aufbeißsensor 58 auf der dem Unterkieferbereich des Patienten zugewandten Seite ebenfalls flächig angeordnete Druckfühler 90, die beim Aufbeißen des Patienten auf den Aufbeißsensor 58 durch dessen Unterkieferzähne niedergedrückt werden und entsprechend mit dem Kieferform-Erkennungskreis 62 kommunizieren.

Die niedergedrückten Druckfühler 90 stehen also für die Lage der erfaßten Zähne zueinander, auf Grund derer der Kieferform-Erkennungskreis 62 ein Kieferform-Signal erzeugt, welches dem Zahnbogen des Patienten bezogen auf den Ober- und den Unterkiefer 24 bzw. 26 entspricht und welches als Grundlage bei der Berechnung der Zahnbogen-Geometrie des Patienten durch die Recheneinheit 32 dient.

Die Recheneinheit 32 kann nun die durch die Bahn 84 der Drehachse A des Panorama-Röntgengerätes 10 vorgegebene Projektions-Bogenfläche, hier sei die in Figur 3A dargestellte Projektionsbogenfläche 88a herausgegriffen, mit der Form des Zahnbogens des Patienten vergleichen und beide Formen aneinander anpassen. Dies geschieht z.B. dadurch, daß das mittlere Abstandsquadrat zwischen Zahnbogen und einem für das Gerät einstellbaren Standard-Projektionsbogen durch Translationen und/oder Rotationen des Zahnbogens minimiert wird (least square fit).

Als Ergebnis schlägt die Recheneinheit 32 Soll-Lagekoordinaten für den Kiefer 24, 26 des Patienten vor, insbesondere Soll-Horizontal-Koordinaten der Kinnauflage 52. Als Ergebnis dieser Anpassung wird, wie es in Figur 3A veranschaulicht ist, der Zahnbogen 70 des Patienten entlang des Pfeiles 92 verfahren, bis die berechneten Soll-Horizontal-Koordinaten erreicht sind und der Zahnbogen 70 des Patienten in einem möglichst großen Bereich mit der Projektionsbogenfläche 88a überlappt. Letzteres ist in Figur 3B veranschaulicht.

Wird der Zahnbogen des Patienten von dem Panorama-Röntgengerät 10 entfernt ermittelt, z.B. anhand eines Gipsabdruckes, berechnet die Recheneinheit 32 Soll-Lage-Koordinaten im Raum bezogen auf das Panorama-Röntgengerät 10.

In Weiterbildung ist es möglich, daß die unterschiedlichen Positionen der einzelnen Komponenten 52, 54 und 56 mittels Servomotoren einstellbar sind, welche Ist-Positionssignale zur Recheneinheit 32 übermitteln und von dieser in Soll-Positionen gesteuert werden. Somit kann auch die resultierende Soll-Relativlage des Patienten automatisch eingestellt werden.

Neben dieser Anpassung der Relativlage des Patienten an eine bestehenden Positionsbogenfläche kann die Recheneinheit 32 die Bahn 84 der Diagnostikeinheit 20 derart berechnen, daß die Projektionsbogenfläche von einer wenig zu den ermittelten Zahnbogen des Patienten passenden Form, wie dies beispielsweise in Figur 4A in Form der Projektionsbogenfläche 88b dargestellt ist, zu einer Projektionsbogenfläche 88c (Figur 4B) verändert wird, die dem Verlauf des Zahnbogens des Patienten in hohem Maße entspricht.

Dieser Vorgang des Anpassens der Projektionsbogenfläche an den Zahnbogen des Patienten wird hier selbständig durch die Recheneinheit 32, den Aufbeißsensor 58 und die verschiedenen Stellungsgeber und gegebenenfalls Servomotoren durchgeführt, die mit den Komponenten 52, 54, 56 zusammenarbeiten, so daß insgesamt eine vollautomatische Abstimmung der Soll-Relativlage des Patienten und insbesondere der Bahn 84 der Drehachse A der Diagnostikeinheit 20 des Panorama-Röntgengerätes 10 erfolgt.

Durch die Druckfühler 90 des Aufbeißsensors 58 werden, wie in Figur 5 erkennbar, neben dem vorderen stark gekrümmten Bereich 74 des Zahnbogens 70 auch der jeweilig schwächer gekrümmte mittlere Bereich 78 bzw. der schwach gekrümmte hintere Bereich 82 erfaßt. Dadurch ist die Zahnbogenform des Patienten mit hoher Genauigkeit entsprechend der tatsächlichen Zahnbogenform des Patienten ermittelbar.

Die Anpassung der Projektionsbogenfläche an die Zahnbogen-Geometrie des Patienten beruht folglich auf dem tatsächlich vorliegenden Zahnbogen des Patienten, so daß eine in hohem Maße auf den Patienten individuell abgestimmte Überlappung der Projektionsbogenfläche 88 des Panorama-Röntgengerätes 10 und dem Zahnbogen 68 oder 70 des Patienten erhalten wird.

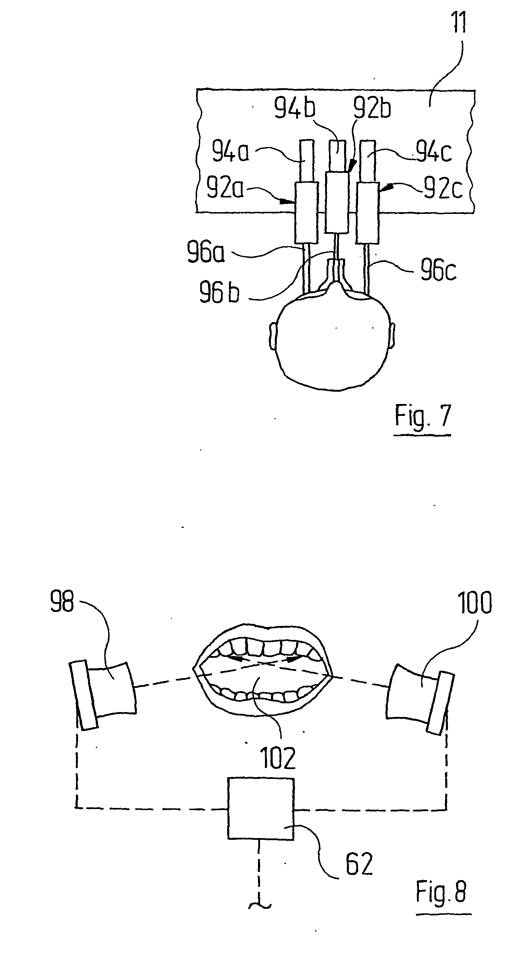

Die Figuren 6 und 7 zeigen ein weiteres Ausführungsbeispiel einer Vorrichtung zur Bestimmung der Soll-Relativlage eines Patienten in einem dentalen Panorama-Röntgengerät bzw. der Soll-Bahn einer Diagnostikeinheit bezüglich des in einem Panorama-Röntgengerät fixierten Kiefers eines Patienten. In diesen Figuren sind dem Ausführungsbeispiel der Figur 1 entsprechende Komponenten mit denselben Bezugszeichen gekennzeichnet.

Abweichend von dem Ausführungsbeispiel der Figur 1 ist auf dem Aufbeißhalter 54 kein Sensor angeordnet.

Die Kieferform-Sensoreinheit 66 umfaßt vielmehr mehrere als elektromechanische Taster ausgebildete Stellungsgeber 92, die über entsprechende Datenübertragungsleitungen 60 mit dem Kieferform-Erkennungskreis 62 kommunizieren. In Figur 7 sind beispielhaft drei nebeneinander angeordnete Stellungsgeber 92a, 92b, 92c in Aufsicht dargestellt.

Die Stellungsgeber 92 sind entlang einer geradlinigen Bahn 94a, 94b bzw. 94c horizontal verfahrbar und in einer gewünschten Position fixierbar. Die Position einer Tastspitze 96 eines Stellungsgebers 92 wird über die Datenübertragungsleitung 60 an den Kieferform-Erkennungskreis 62 übermittelt.

Zur Ermittlung der Kieferform des Patienten wird die Kieferform-Sensoreinheit 66 bei in der Positioniervorrichtung 50 fixierten Patienten in Richtung auf den Patienten bewegt, bis die Tastspitzen 96 der Stellungsgeber 92 den Kopf 22 des Patienten äußerlich in Höhe kurz oberhalb der Oberlippe berühren. Dazu ist der Patient vorher in eine entsprechende geeignete vertikale Position zu bringen.

Die Ausgangssignale der Stellungsgeber 92 werden auf den Kieferform-Erkennungskreis 62 gegeben. Dieser wiederum erzeugt ein Kieferform-Signal, welches einem Muster-Zahnbogen entspricht, welcher zu den Positionen der Stellungsgeber 92 paßt und welcher dem Zahnbogen des Patienten nahekommt.

Nachdem die Recheneinheit 32 das Kieferform-Signal über die Datenübertragungsleitung 64 empfangen hat, berechnet sie die Soll-Relativlage des Patienten ähnlich wie oben beschrieben, z.B. durch least square fit. Das weitere zu dem ersten Ausführungsbeispiel nach den Figuren 1 bis 5 Gesagte gilt für das zweite Ausführungsbeispiel der Figuren 6 und 7 sinngemäß entsprechend.

Durch Erhöhung der Anzahl der Stellungsgeber 92 kann die Genauigkeit der Bestimmung der Zahnbogen-Geometrie des Patienten erhöht werden.

Figur 8 zeigt schematisch ein weiteres Ausführungsbeispiel, bei dem dem Ausführungsbeispiel der Figur 1 entsprechende Komponenten dieselbe Bezugszeichen tragen.

Bei diesem Ausführungsbeispiel wird die Soll-Relativlage des Patienten zu dem Panorama-Röntgengerät ermittelt, in dem die Zahnbogen-Geometrie des Patienten mittels zweier Kameras 98 und 100 bestimmt wird, die den Mundraum 102 und damit die Zähne des Patienten aus zwei unterschiedlichen Winkeln erfassen. Die Signale der Kameras 98, 100 werden über entsprechende Datenübertragungsleitungen 104 und 106 an den Kieferform-Erkennungskreis 62 übertragen, welcher einen geeigneten Bildverarbeitungsalgorithmus ausführt, der als Ergebnis ein Kieferform-Signal an die Recheneinheit 32 sendet, aus dem diese die Zahnbogen-Geometrie des Patienten berechnet.

Die weitere Berechnung der Soll-Relativlage des Patienten oder der Soll-Bahn der Diagnostikeinheit 20 des Panorama-Röntgengerätes 10 erfolgt wieder entsprechend den oben erwähnten Vorgängen.

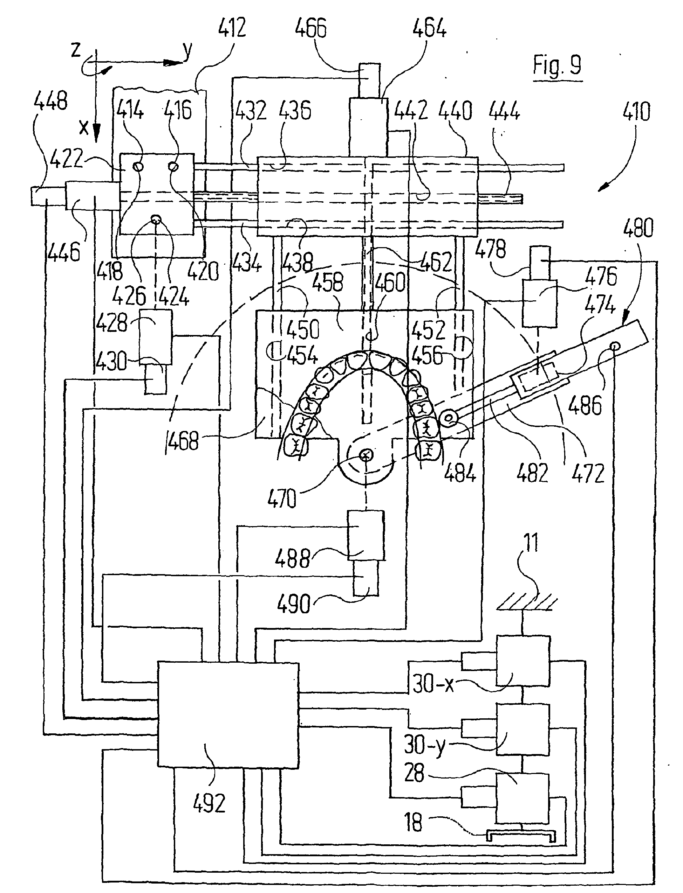

Figur 9 zeigt eine insgesamt mit 410 bezeichnete Vorrichtung, die dazu dient, eine Diagnostikeinheit 20 eines Panorama-Röntgengerätes 10, welche die Röntgenstrahlungsquelle 14, den CCD-Sensor 16 und den Dreharm 18 umfaßt, so in der Aufnahmeebene zu führen, daß der Projektionsbogen 88 des Röntgengerätes 10 mit dem Zahnbogen 68, 70 des zu untersuchenden Patienten übereinstimmt.

Von einem feststehenden Abschnitt 412 des Rahmens des Röntgengerätes sind zwei beabstandete vertikale Führungsstäbe 414, 416 getragen, die mit Führungsbohrungen 418, 420 eines z-Schlittens 422 zusammenarbeiten. Im letzteren ist ferner eine Gewindebohrung 424 vorgesehen, in welcher eine Gewindespindel 426 läuft. Letztere wird durch einen Elektromotor 428 angetrieben, der mit einem Stellungsgeber 430 gekoppelt ist. Dieser kann z.B. eine Stroboskopscheibe umfassen, die mit einer Lichtschranke zusammenarbeitet, wobei der Ausgang der Lichtschranke mit einem Zähler verbunden ist. Das Ausgangssignal des Stellungsgebers 430 ist somit eindeutig mit der Stellung des z-Schlittens 422 verbunden.

Der z-Schlitten 422 trägt seinerseits zwei Führungsstäbe 432, 434, die in Bohrungen 436, 438 eines y-Schlittens 440 laufen. Letzterer ist ferner mit einer Gewindebohrung 442 versehen, in welcher eine Gewindespindel 444 läuft. Diese wird durch einen Elektromotor 446 angetrieben, der mit einem Stellungsgeber 448 verblockt ist. Der Stellungsgeber 448 ist genauso aufgebaut wie der Stellungsgeber 430.

Der y-Schlitten 440 trägt zwei Führungsstäbe 450, 452, welche in Bohrungen 454, 456 eines x-Schlittens 458 laufen. Dieser ist mit einer Gewindebohrung 460 versehen, in welcher eine Gewindespindel 462 läuft. Letztere wird durch einen Elektromotor 464 angetrieben, an den wieder ein Stellungsgeber 466 angeflanscht ist.

Der x-Schlitten 458 dient als Abstützung für das Kinn eines Patienten und ist mit einer dünnen nachgiebigen Auflage 468 versehen.

Aus der vorstehenden Beschreibung ist ersichtlich, daß durch die verschiedenen Schlitten und die auf sie arbeitenden Elektromotoren die Auflage 468 in x-, y- und z-Richtung eingestellt werden kann. An der Unterseite des x-Schlitten 458 ist eine Welle 470 gelagert, die fest mit einem Schwenkarm 472 verbunden ist. Der Schwenkarm 472 trägt an der Oberseite seines äußersten Abschnittes eine längenveränderliche Strebe 474, die sich in z-Richtung erstreckt und durch einen zugeordneten Elektromotor 476 in der Länge verstellt werden kann. Der Elektromotor 476 ist wieder mit einem Stellungsgeber 478 verblockt.

Das obere Ende der Strebe 474 trägt einen Tastkopf 480, der nach Art eines Federzylinders ausgebildet ist. Ein durch die Feder vorgespannter Taststab 482 trägt an seinem freien Ende eine freilaufende Rolle 484, welche um eine vertikale Achse umläuft. Die axiale Abmessung der Rolle 484 ist so bemessen, daß sie etwas kleiner ist als die mittlere Höhe durchschnittlicher Zähne. Der Durchmesser der Rolle 484 ist so bemessen, daß sie gut auf der Außenseite des den Zahnbogen überdeckenden Weichgewebes läuft.

Der Tastkopf 480 umfaßt ferner einen Stellungsgeber 486, der die Stellung des Taststabes 482 ermittelt.

Zum Verschwenken des Tastkopfes 480 über den Zahnbogen 68 hinweg ist mit der Welle 460 ein Elektromotor 488 gekoppelt, der mit einem Stellungsgeber 490 verblockt ist.

Die verschiedenen Stellungsgeber 430, 448, 466, 478 und 490 sind jeweils identisch aufgebaut. Der Stellungsgeber 486 kann im Prinzip ähnlichen Aufbau haben, wenn seine Eingangswelle mit einem Ritzel versehen ist, welches mit einer auf den Taststab 482 ausgebildeten Zahnstange zusammenarbeitet. Der Stellungsgeber 486 kann alternativ auch eine Lichtschranke umfassen, die einen durch den Taststab 482 bewegten Strichgitter zusammenarbeitet.

Die Ausgangssignale der verschiedenen Stellungsgeber sind mit Eingängen einer Steuereinheit 492 verbunden, die ferner Ausgänge hat, an denen Steuersignale für die Elektromotoren 428, 446, 464, 476 und 488 bereitgestellt werden.

Mit der in Figur 9 gezeigten Vorrichtung kann die Ist-Lage und die Ist-Form des Zahnbogens 68 in einem Panorama-Röntgengerät 10 gemessen werden.

Hierzu wird zunächst durch entsprechende Ansteuerung der Elektromotoren 428, 446 und 464 die Auflage 468 so gestellt, daß der Zahnbogen in der Aufnahmeebene E liegt.

In dieser wird der Kopf des Patienten dann fixiert. Durch Ansteuerung des Elektromotors 476 wird dann die Strebe 474 so weit ausgefahren, daß die Rolle 484 des Tastkopfes 480 in gleicher Höhe liegt wie der zu vermessende Zahnbogen bzw. Kiefer.

Nun wird der Elektromotor 488 so erregt, daß der Schwenkarm 472 um 180° gedreht wird. Bei dieser Bewegung folgt die Rolle 484 unter der Kraft der Vorspannfeder des Tastkopfes 480 der Außenkontur des Zahnbogens bzw. des Kiefers.

Die entsprechenden Ausgangssignale des Stellungsgebers 486 werden zusammen den Ausgangssignalen des Stellungsgebers 490 in der Steuereinheit 492 gespeichert.

Diese enthält somit dann in digitaler Form die Ist-Außenkontur und Ist-Lage des Kiefers bzw. Zahnbogens. Über die Ausgangssignale der Stellungsgeber 448 und 466 kennt die Steuereinheit 492 auch die Absolutlage des Zahnbogens in der x-y-Ebene, und durch Addieren der Ausgangssignale des Stellungsgebers 430 und des Stellungsgebers 478 kennt man auch die z-Koordinate des Zahnbogens. Diese sollte der z-Koordinate der Aufnahmeebene E entsprechen.

Bei einer vereinfachten Ausführungsform der Vorrichtung kann man den z-Schlitten 422 und den y-Schlitten 440 auch weglassen und nur den Schlitten 458 vorsehen, der dann nur in vertikaler Richtung einstellbar ist, sei es manuell, sei es durch einen Servomotor.

Die Steuereinheit 492 berechnet aus dem wie oben beschrieben gemessenen Zahnbogen diejenige Bahn 84, auf welcher die Achse A in der x-y-Ebene bewegt werdem muß, damit die Projektionsbogenfläche 88 des Panorama-Röntgengerätes 10 mit dem gemessenen Zahnbogen zusammenfällt. Entsprechende Steuersignale werden auf den in x-Richtung wirkenden Koordinatenantrieb 30-x und den in y-Richtung wirkenden Koordinatenantrieb 30-y gegeben, welche zusammen die Lage der Achse A in der x-y-Ebene einstellen.

Die Materialien der im Strahlungsweg zwischen Röntgenstrahlungsquelle und CCD-Sensor 16 befindlichen Komponenten, d.h. der Positioniervorrichtung 50 sowie der Sensoren 58, 92 und 98, 100 sind im wesentlichen aus Werkstoffen, welche eine geringe Röntgenabsorption aufweisen, insbesondere Kunststoffmaterial.

Der bei den vorgenannten Ausführungsbeispielen angesprochene Kieferform-Erkennungskreis 62 kann auch direkt ein Bestandteil der entsprechenden Recheneinheit 32 bzw. ein in dieser laufendes Programm sein, so daß die jeweiligen Sensoren 58, 92 und 98, 100 ihre jeweiligen Ausgangssignale direkt an die Recheneinheit 32 übertragen.

Die Sensoren 58, 92 und 98, 100 sind auch dazu geeignet, den Zahnbogen eines Patienten zu ermitteln, ohne daß dieser in der Positioniervorrichtung 50 fixiert ist. Erst nach der Bestimmung seines Zahnbogens außerhalb des Panorama-Röntgengerätes 10 wird der Patient dann in die entsprechend berechnete Soll-Relativlage gebracht.

Auch ist eine Bestimmung des Zahnbogens durch die Sensoren 58, 92 und 98, 100 möglich, ohne daß der Patient anwesend sein muß. Dies gilt beispielsweise, wenn ein Kieferabdruck von dem Kiefer des Patienten vorliegt, welcher dann von dem entsprechenden Sensor wie ein natürlicher Kiefer erfaßt und vermessen werden kann.

## Patentansprüche

1. Verfahren zur Bestimmung der Soll-Relativlage eines Patienten in einem dentalen Panorama-Röntgengerät,
bei welchem
a) die Krümmung eines vorderen Bereichs (72, 74) eines Zahnbogens (68, 70) eines Patienten ermittelt wird; und
b) daraus und aus einer Projektionsbogenfläche (88) des Panorama-Röntgengerätes (10) Soll-Lagekoordinaten für den Kiefer (24, 26) des Patienten berechnet werden,
**dadurch gekennzeichnet, daß**
c) zusätzlich die Krümmung eines mittleren Bereichs (76, 78) und/oder hinteren Bereichs (80, 82) des Zahnbogens (68, 70) des Patienten ermittelt wird und bei der Berechnung der Soll-Lagekoordinaten für den Kiefer (24, 26) berücksichtigt wird.

2. Verfahren zur Bestimmung der Soll-Bahn, auf welcher
ein dentales Panorama-Röntgengerät bezüglich eines Patienten bewegt wird, bei welchem
a) die Krümmung eines vorderen Bereichs (72, 74) eines Zahnbogens (68, 70) eines Patienten ermittelt wird; und
b) der Zahnbogen (68, 70) des Patienten an einer Stelle im Panorama-Röntgengerät (10) fixiert wird, die innerhalb des von einer Röngenstrahlungsquelle (14) und einer starr mit dieser verbundenen Detektoreinheit (16) überstrichenen Raumes liegt,
**dadurch gekennzeichnet, daß**
c) zusätzlich die Krümmung eines mittleren Bereichs (76, 78) und/oder hinteren Bereichs (80, 82) des Zahnbogens des Patienten ermittelt wird; und
d) aus den gemessenen Krümmungen des vorderen (72, 74) und mittleren (76, 78) und/oder hinteren Bereichs (80, 82) des Zahnbogens (68, 70) eine Soll-Bahnkurve für die durch Röngtenstrahlungsquelle (14) und Detektoreinheit (16) gebildete Diagnostikeinheit (20) berechnet wird.

3. Vorrichtung zur Bestimmung der Soll-Relativlage
eines Patienten in einem dentalen Panorama-Röntgengerät bzw. der Soll-Bahn einer Diagnostikeinheit (20) bezüglich des in einem Panorama-Röntgengerät fixierten Kiefers (24, 26) eines Patienten, mit
a) einer Sensoreinheit (66), welche derart ausgebildet ist, dass sie mit Zähnen des Patienten im vorderen Bereich (72, 74) des Kiefers (24, 26) zusammenarbeitet und welche ein für die Lage der Zähne des Patienten zueinander charakteristisches Kieferform-Signal bereitstellt; und
b) einer Recheneinheit (32), welche derart ausgebildet ist, dass sie mit dem Kieferform-Signal der Sensoreinheit (66) beaufschlagt ist und aus diesem und der Bahn (84) einer Röntgenstrahlungsquelle (14) und einer Detektoreinheit (16) des Panorama-Röntgengerätes (10), die die Projektionsbogenfläche (88) des Panorama-Röntgengeräts (10) bestimmt, Soll-Lagekoordinaten für den Kiefer (24, 26) des Patienten berechnet,
wobei
c) die Sensoreinheit (66) derart ausgebildet ist, dass sie zusätzlich mit den Zähnen des Patienten im mittleren Bereich (76, 78) und/oder hinteren Bereich (80, 82) des Kiefers (24, 26) zusammenarbeitet.

4. Vorrichtung nach Anspruch 3, **dadurch gekennzeichnet,**
**daß** die Sensoreinheit (66) wenigstens einen Sensor (58; 92; 98, 100; 480) und einen damit kommunizierenden Kiefer-Erkennungskreis (62) umfaßt, der ein dem Sensor-Ausgangssignal ensprechendes für die Zahnbogen-Geometrie des Patienten charakteristisches Kieferform-Signal erzeugt.

5. Vorrichtung nach Anspruch 3 oder 4, **dadurch gekennzeichnet, daß** die Sensoreinheit (66) einen Aufbeißsensor (58) umfaßt, welcher an einem Aufbeißhalter (54) des Panorama-Röntgengerätes (10) angeordnet ist.

6. Vorrichtung nach Anspruch 5, **dadurch gekennzeichnet,**
**daß** der Aufbeißsensor (58) auf einer dem Oberkiefer (24) und/oder dem Unterkiefer (26) des Patienten zugewandten Seite eine Mehrzahl von Druckfühlern (90) umfaßt.

7. Vorrichtung nach Anspruch 3 oder 4, **dadurch gekennzeichnet, daß** die Sensoreinheit (66) eine verfahrbare Tastspitze (96; 484) und eine deren Stellung angebende Stellungsgeber-Anordnung (92a, 92b, 92c; 486) umfaßt.

8. Vorrichtung nach Anspruch 7, **dadurch gekennzeichnet,**
**daß** der Sensor (480) in zur Tastrichtung senkrechter Richtung verfahrbar (470, 472) ist und ein weiterer Stellungsgeber (490) die Sensorstellung in Verfahrrichtung ermittelt.

9. Vorrichtung nach Anspruch 3 oder 4, **dadurch gekennzeichnet, daß** die Sensoreinheit (66) mindestens eine Kamera (98, 100) umfaßt, welche derart angeordnet ist, daß sie ein Bild des Kiefers (24; 26) des Patienten erstellt.

10. Vorrichtung nach einem der Ansprüche 3 bis 9, **dadurch gekennzeichnet, daß** die Sensoreinheit (66) im wesentlichen aus Werkstoffen besteht, welche eine geringe Röntgenabsorption aufweisen.

11. Vorrichtung nach einem der Ansprüche 4 bis 10, **dadurch gekennzeichnet, daß** der Kiefer-Erkennungskreis (62) in die Recheneinheit (32) integriert ist.

## Claims

1. Method for determining the setpoint relative position of a patient in a dental panorama X-ray apparatus, in which
a) the curvature of a front region (72, 74) of a patient's dental arch (68, 70) is determined; and
b) setpoint position coordinates for the patient's jaw (24, 26) are calculated from this and from a projection arch surface (88) of the panorama X-ray apparatus (10),
**characterised in that**
c) the curvature of a central region (76, 78) and/or rear region (80, 82) of the patient's dental arch (68, 70) is additionally determined and taken into account in the calculation of the setpoint position coordinates for the jaw (24, 26).

2. Method for determining the setpoint path on which a dental panorama X-ray apparatus is moved relative to a patient, in which
a) the curvature of a front region (72, 74) of a patient's dental arch (68, 70) is determined; and
b) the patient's dental arch (68, 70) is fixed at a position in the panorama X-ray apparatus (10) which lies within the space scanned by an X-ray source (14) and a detector unit (16) rigidly connected to it,
**characterised in that**
c) the curvature of a central region (76, 78) and/or rear region (80, 82) of the patient's dental arch is additionally determined; and
d) a setpoint trajectory for the diagnostic unit (20) formed by the X-ray source (14) and the detector unit (16) is calculated from the measured curvatures of the front region (72, 74) and central region (76, 78) and/or rear region (80, 82) of the dental arch (68, 70).

3. Device for determining the setpoint relative position of a patient in a dental panorama X-ray apparatus or the setpoint path of a diagnostic unit (20) relative to a patient's jaw (24, 26) fixed in a panorama X-ray apparatus, having
a) a sensor unit (66) which is adapted so that it interacts with teeth of the patient in the front region (72, 74) of the jaw (24, 26) and which provides a jaw shape signal characteristic of the position of the patient's teeth relative to one another; and
b) a computing unit (32) which is adapted so that it receives the jaw shape signal of the sensor unit (66) and calculates setpoint position coordinates for the patient's jaw (24, 26) from this and from the path (84) of an X-ray source (14) and a detector unit (16) of the panorama X-ray apparatus (10), which detector unit determines the projection arch surface (88) of the panorama X-ray apparatus (10),
wherein
c) the sensor unit (66) is adapted so that it additionally interacts with the patient's teeth in the central region (76, 78) and/or rear region (80, 82) of the jaw (24, 26).

4. Device according to claim 3, **characterised in that** the sensor unit (66) comprises at least one sensor (58; 92; 98, 100; 480) and a jaw recognition circuit (62) which communicates therewith and generates a jaw shape signal which corresponds to the sensor output signal and is characteristic of the patient's dental arch geometry.

5. Device according to claim 3 or 4, **characterised in that** the sensor unit (66) comprises a bite sensor (58) arranged on a bite holder (54) of the panorama X-ray apparatus (10).

6. Device according to claim 5, **characterised in that** the bite sensor (58) comprises a multiplicity of pressure pickups (90) on a side facing the patient's upper jaw (24) and/or lower jaw (26).

7. Device according to claim 3 or 4, **characterised in that** the sensor unit (66) comprises a displaceable sampling tip (96; 484) and a position transducer arrangement (92a, 92b, 92c; 486) indicating its position.

8. Device according to claim 7, **characterised in that** the sensor (480) can be displaced (470, 472) in a direction perpendicular to the sampling direction and a further position transducer (490) determines the sensor's position in the displacement direction.

9. Device according to claim 3 or 4, **characterised in that** the sensor unit (66) comprises at least one camera (98, 100) which is adapted so that it takes a picture of the patient's jaw (24; 26).

10. Device according to any one of claims 3 to 9, **characterised in that** the sensor unit (66) substantially consists of materials which have low X-ray absorption.

11. Device according to any one of claims 4 to 10, **characterised in that** the jaw recognition circuit (62) is integrated into the computing unit (32).

## Revendications

1. Procédé pour déterminer la position relative théorique d'un patient dans un appareil radiographique panoramique dentaire, dans lequel
a) la courbure d'une région antérieure (72, 74) d'une arcade dentaire (68, 70) d'un patient est déterminée ; et
b) à partir de cette courbure et d'une surface d'arc de projection (88) de l'appareil radiographique panoramique (10) sont calculées des coordonnées de position théorique pour la mâchoire (24, 26) du patient,
**caractérisé en ce que**
c) la courbure d'une région médiane (76, 78) et/ou d'une région postérieure (80, 82) de l'arcade dentaire (68, 70) du patient est également déterminée et prise en compte dans le calcul des coordonnées de position théorique pour la mâchoire (24, 26).

2. Procédé pour déterminer la trajectoire théorique sur laquelle un appareil radiographique panoramique dentaire est déplacé par rapport à un patient, dans lequel
a) la courbure d'une région antérieure (72, 74) d'une arcade dentaire (68, 70) d'un patient est déterminée ; et
b) l'arc dentaire est fixé en un point de l'appareil radiographique panoramique qui se trouve à l'intérieur de l'espace balayé par une source de rayons X (14) et une unité de détection (16) reliée rigidement à celle-ci,
**caractérisé en ce que**
c) la courbure d'une région médiane (76, 78) et/ou d'une région postérieure (80, 82) de l'arcade dentaire du patient est également déterminée ; et
d) une courbe de trajectoire théorique pour l'unité de diagnostic (20) formée par la source de rayons X (14) et l'unité de détection (16) est calculée à partir des courbures mesurées des régions antérieure (72, 74), médiane (76, 78) et/ou postérieure (80, 82) de l'arcade dentaire (68, 70).

3. Dispositif pour déterminer la position relative théorique d'un patient dans un appareil radiographique panoramique dentaire ou la trajectoire théorique d'une unité de diagnostic (20) par rapport à la mâchoire (24, 26) d'un patient fixée dans un appareil radiographique panoramique, avec
a) une unité de capteur (66), laquelle est conçue de manière à coopérer avec des dents du patient dans la région antérieure (72, 74) de la mâchoire (24, 26) et laquelle fournit un signal de forme de mâchoire caractéristique de la position des dents du patients les unes par rapport aux autres ; et
b) une unité de calcul (32), laquelle est conçue de manière à recevoir le signal de forme de mâchoire de l'unité de capteur (66) et à calculer à partir de celui-ci et de la trajectoire (84) d'une source de rayons X (14) et d'une unité de détection (16) de l'appareil radiographique panoramique (10), qui détermine la surface d'arc de projection (88) de l'appareil radiographique panoramique (10), des coordonnées de position théorique pour la mâchoire (24, 26) du patient,
c) l'unité de capteur (66) étant en outre conçue de manière à coopérer avec les dents du patient dans la région médiane (76, 78) et/ou la région postérieure (80, 82) de la mâchoire (24, 26).

4. Dispositif selon la revendication 3, **caractérisé en ce que** l'unité de capteur (66) comprend au moins un capteur (58 ; 92 ; 98, 100 ; 480) et un circuit de reconnaissance de mâchoire (62) communiquant avec celui-ci, qui génère un signal de forme de mâchoire caractéristique de la géométrie de l'arcade dentaire du patient correspondant au signal de sortie du capteur.

5. Dispositif selon la revendication 3 ou 4, **caractérisé en ce que** l'unité de capteur (66) comprend un capteur à mordre (58), lequel est disposé sur un support à mordre (54) de l'appareil radiographique panoramique (10).

6. Dispositif selon la revendication 5, **caractérisé en ce que** le capteur à mordre (58) comprend une pluralité de capteurs de pression (90) sur un côté tourné vers la mâchoire supérieure (24) et/ou la mâchoire inférieure (26) du patient.

7. Dispositif selon la revendication 3 ou 4, **caractérisé en ce que** l'unité de capteur (66) comprend une pointe de palpage déplaçable (96 ; 484) et un dispositif transmetteur de position (92a, 92b, 92c ; 486) qui indique sa position.

8. Dispositif selon la revendication 7, **caractérisé en ce que** le capteur (480) est déplaçable dans une direction (470, 472) perpendiculaire à la direction de palpage et un transmetteur de position supplémentaire (490) détermine la position du capteur dans la direction de déplacement.

9. Dispositif selon la revendication 3 ou 4, **caractérisé en ce que** l'unité de capteur (66) comprend au moins une caméra (98, 100), laquelle est disposée de manière à réaliser une image de la mâchoire (24 ; 26) du patient.

10. Dispositif selon une des revendications 3 à 9, **caractérisé en ce que** l'unité de capteur (66) est composée essentiellement de matériaux qui présentent une faible absorption des rayons X.

11. Dispositif selon une des revendications 4 à 10, **caractérisé en ce que** le circuit de reconnaissance de mâchoire (62) est intégré dans l'unité de calcul (32).
